# EUROPÄISCHE PATENTSCHRIFT

(11) **EP 4 093 982 B1**
(45) Veröffentlichungstag und Bekanntmachung des Hinweises auf die Patenterteilung: **20.12.2023**
(21) Anmeldenummer: 21701438.0
(22) Anmeldetag: 19.01.2021
(51) Int. Cl.: F16C 19/06, F16C 33/58, F16C 33/62, F16C 33/64, F16C 35/067, F16C 41/00, F16C 19/54, A61B 17/16

(54) **SIGNALWEITERLEITUNG BZW. -ÜBERTRAGUNG IN EINEM CHIRURGISCHEN INSTRUMENT**
SIGNAL FORWARDING OR TRANSMISSION IN A SURGICAL INSTRUMENT
TRANSFERT OU TRANSMISSION DE SIGNAL DANS UN INSTRUMENT CHIRURGICAL

(30) Priorität: 20.01.2020 DE 102020101171
(43) Veröffentlichungstag der Anmeldung: 30.11.2022
(73) Patentinhaber: Aesculap AG, 78532 Tuttlingen (DE)
(72) Erfinder: HÖGERLE, Roland-Alois, 78532 Tuttlingen (DE); LENZENHUBER, Frederick, 78532 Tuttlingen (DE); BÜRK, André, 78056 Villingen-Schwenningen (DE)
(74) Vertreter: Winter, Brandl - Partnerschaft mbB
(86) Internationale Anmeldenummer: PCT/EP2021/051061
(87) Internationale Veröffentlichungsnummer: WO 2021/148400

(56) Entgegenhaltungen:
- EP-A1- 2 589 347
- WO-A1-2014/116782
- CN-U- 204 394 449
- DE-A1-102008 016 592
- DE-A1-102015 007 593
- DE-T2- 69 808 255
- US-A1- 2014 074 084
- US-A1- 2018 064 438

## Beschreibung

Die vorliegende Erfindung betrifft eine Signalweiterleitung bzw. -übertragung in einem chirurgischen Instrument, beispielsweise einem Fräshandstück. Insbesondere betrifft die vorliegende Erfindung ein chirurgisches Instrument mit einem Wälzlager, ein Verfahren zur Herstellung eines Wälzlagers und eine Verwendung eines Wälzlagers in einem chirurgischen Instrument.

### Stand der Technik

Chirurgische Motorensysteme, das heißt chirurgische Instrumente, welche einen Motor aufweisen, wie etwa Fräshandstücke, werden zunehmend mit neuen Funktionen ausgestattet, die eine Übermittlung, Weiterleitung bzw. Übertragung von elektrischen Signalen erfordern. Derartige neue Funktionen können beispielsweise sein, dass eine Temperatur an einer Spitze des Fräshandstücks über Temperatursensoren ermittelt wird, dass Kräfte beim Fräsen über Dehnungsmessstreifen ermittelt werden, oder dass ein in das Fräshandstück eingesteckter Werkzeugtyp über Sensoren oder Antennen, beispielsweise RFID respektive NFC-Ausleseantennen, identifiziert wird. Diese neuen Funktionen haben gemeinsam, dass sie für eine Übermittlung, Weiterleitung bzw. Übertragung von Daten eine elektrische Verbindung zu einem Steuergerät benötigen.

Eine solche elektrische Verbindung wird bisher beispielsweise durch Anbringen bzw. Einfügen von Signalleitungen in das Fräshandstück realisiert. Dabei werden elektrische Signale über einzelne, isolierte Signallitzen übertragen, welche in einem separaten Kanal durch den Schaft des Fräshandstücks verlaufen und sich bis hin zu einer Spitze/ einem distalen Ende des Fräshandstücks erstrecken. Aufgrund der kompakten Bauart und des begrenzten Bauraums von chirurgischen Fräshandstücken bedeutet ein Einbringen bzw. Einfügen von herkömmlichen Signalleitungen in das Fräshandstück jedoch, dass ein Außendurchmesser eines Schaftes des Fräshandstücks vergrößert werden muss, da ein zusätzlicher Kanal für die isolierten Signallitzen erforderlich wird.

Diese mangelnde Integration der Signallitzen in den bestehenden Aufbau der Fräshandstücke ist aus Anwendersicht jedoch nicht wünschenswert und als negativ zu beurteilen, da sich dadurch ein Sichtzugang für den Anwender/ Chirurg verschlechtert und das Handstück seine Eignung für enge Zugänge verliert. Weiterhin zeichnet sich der bestehende Aufbau durch eine erschwerte Montage, schwierige Anschlussmöglichkeiten und eine komplizierte Anbindung von mehreren Signalgebern bei einer Verwendung als Bus-System aus.

Die DE 10 2015 007593 A1 offenbart ein Radlager für ein Fahrzeug mit Heizelementen, die über elektrische Versorgungsleitungen mit elektrischer Energie versorgt werden. Die Heizelemente dienen einem (Auf-)Heizen des Radlagers und sind vorgesehen, um eine Lagerreibung und einen Lagerverschleiß zu verringern.

Die DE 10 2008 016592 A1 offenbart ein Messlager für einen Radsatz eines Schienenfahrzeugs, welches die tatsächlichen Belastungen des Lagers zuverlässig misst. Dabei sind als Dehnungsmessstreifen ausgebildete Sensoren vorgesehen, sowie Signalleitungen, welche in Nuten geführt sind.

Die EP 2 589 347 A1 offenbart ein chirurgisches Greifinstrument mit einem hohlzylindrischen Bauteil, durch welches Signalleitungen laufen. Ferner offenbart die DE 698 08 255 T2 eine Hülle, die an einem chirurgischen Instrument befestigt werden kann und Leiter zur Übertragung von elektrischen Signalen aufweist. Außerdem offenbart die CN 204 394 449 U eine Buchse bzw. Hülse, welche zur Bilderfassung in vivo in Tierversuchen vorgesehen ist und Elektroden zur Signalerfassung/ -übertragung/ -weiterleitung durch die Hülse aufweist. Die US 2018/064438 A1, die WO 2014/116782 A1 und die US 2014/074084 A1 offenbaren jeweils hülsenförmige Bauteile, durch welche Kabel/ Signalleitungen laufen.

### Kurzbeschreibung der Offenbarung

Vor diesem Hintergrund ist es Aufgabe der vorliegenden Erfindung, die Nachteile der bestehenden Lösungen zu vermeiden bzw. wenigstens zu mildern. Insbesondere sollen Signalleitungen bzw. Signalbahnen besser in den bestehenden Aufbau von chirurgischen (Motor-) Instrumenten wie etwa Fräshandstücken integriert werden, ohne dabei die Abmessungen des chirurgischen Instruments bzw. der in dem Instrument vorgesehenen Bauteile zu verändern, also ohne beispielsweise den Außendurchmesser des chirurgischen Instruments zu vergrößern.

Offenbarungsgemäß wird eine neue Bauweise eines Wälzlagers und einer Hülse bereitgestellt, welche eine Weiterleitung/ Übertragung/ Übermittlung von elektrischen Signalen durch das Wälzlager und die Hülse hindurch, sowie zwischen diesen Bauteilen ermöglicht, wobei die Abmessungen der Bauteile (Wälzlager und Hülse) unverändert bleiben, so dass der Außendurchmesser des chirurgischen Instruments nicht vergrößert wird. Das chirurgische Instrument behält demnach seine kompakte Bauart und der bestehende Bauraum wird geeignet ausgenutzt.

Die voranstehend genannte Aufgabe wird entsprechend durch ein chirurgisches Instrument nach Anspruch 1, ein Verfahren zur Herstellung eines Wälzlagers nach Anspruch 14 und eine Verwendung eines Wälzlagers nach Anspruch 15 gelöst. Vorteilhafte Ausführungsformen und Weiterbildungen sind in den Unteransprüchen beansprucht und/oder werden nachfolgend erläutert.

Die Offenbarung betrifft zunächst ein Wälzlager, insbesondere ein Kugellager, welches zur, vorzugsweise multidirektionalen, Weiterleitung bzw. Übertragung von elektrischen Signalen eingerichtet ist und dazu zumindest eine in das Kugellager integrierte Signalleitung bzw. Signalbahn aufweist.

Die Offenbarung betrifft grundsätzlich ein Wälzlager und ist daher nicht auf ein Kugellager beschränkt, das heißt es sollen auch beliebige andere Wälzlager wie etwa Zylinderrollenlager, Nadellager, Kegelrollenlager, Tonnenlager, Toroidalrollenlager, etc. von der Offenbarung umfasst sein. Das Kugellager stellt vorliegend jedoch die bevorzugte Ausführungsform eines Wälzlagers gemäß der vorliegenden Offenbarung dar. Weiter bevorzugt ist das Kugellager ein Mikro-Kugellager.

Das Wälzlager/ Kugellager ist bevorzugt zur Verwendung in einem chirurgischen (Motor-) Instrument, insbesondere einem chirurgischen Handstück, insbesondere einem Fräshandstück, eingerichtet bzw. geeignet bzw. vorgesehen.

Es versteht sich daher von selbst, dass die Offenbarung alternativ oder zusätzlich auch eine Verwendung eines Wälzlagers/ Kugellagers in einem chirurgischen Instrument betrifft.

Es ist von Vorteil, wenn die Signalleitung bzw. Signalbahn in einem Außenring des Wälzlagers/ Kugellagers integriert ist.

Bevorzugt ist das Wälzlager/ Kugellager (insbesondere der Außenring des Wälzlagers/ Kugellagers) aus einem nichtleitenden Material hergestellt. Weiter bevorzugt handelt es sich bei dem Material des Wälzlagers/ Kugellagers (des Außenrings) um ein hartes Material. Als besonders geeignet hat sich dabei Keramik herausgestellt.

Die Signalleitung bzw. Signalbahn ist bevorzugt aus einem (gut) leitenden Material, insbesondere Kupfer, Silber oder Gold, hergestellt.

Ein vorteilhaftes Ausführungsbeispiel zeichnet sich dadurch aus, dass zumindest eine Signalleitung, insbesondere Signallitze, vorgesehen ist, welche in eine in dem Wälzlager/ Kugellager, insbesondere dem Außenring des Wälzlagers/ Kugellagers, vorgesehene Bohrung, die sich über eine gesamte axiale Länge des Wälzlagers/ Kugellagers erstreckt, eingesetzt ist. Bevorzugt ist die Signalleitung bzw. Signallitze in der Bohrung axial fixiert.

Dementsprechend weist das Wälzlager/ Kugellager bzw. der Außenring des Wälzlagers/ Kugellagers bevorzugt zumindest eine feine Bohrung auf. Beispielsweise kann der Durchmesser der Bohrung kleiner als 0,2 mm sein. Bevorzugt ist der Durchmesser etwa im Bereich von 0,1 mm. Als Herstellungs-/ bzw. Fertigungsverfahren für eine derart feine Bohrung hat sich insbesondere Mikrolaserbohren als geeignet herausgestellt.

Entsprechend ist auch der Durchmesser der Signalleitung bzw. Signallitze bevorzugt kleiner als 0,2 mm, weiter bevorzugt etwa im Bereich von 0,1 mm.

Ein axiales Fixieren der Signalleitung bzw. Signallitze in der Bohrung kann beispielsweise über ein plastisches Verformen von axialen Enden der Signalleitung bzw. Signallitze realisiert werden. Hier hat sich insbesondere ein Verstemmen als geeignet herausgestellt. Alternativ kann die Bohrung auch zunächst metallisiert werden (bevor die Signalleitung bzw. Signallitze eingesetzt wird) und die axiale Fixierung kann über eine Klebeverbindung oder eine Hartlotverbindung erfolgen.

Von besonderem Vorteil ist es auch, wenn die Signalleitung in axialer Richtung des Wälzlagers/ Kugellagers (über den Außenring) hervorsteht/ heraussteht, insbesondere an beiden Seiten/ axialen Enden des Wälzlagers/ Kugellagers, so dass die Signalleitung zur Kontaktierung bzw. Steckverbindung mit einem anderen Bauteil des chirurgischen Instruments, insbesondere mit einer Hülse, eingerichtet ist. Bevorzugt steht die Signalleitung etwa 0,1 bis 0,5 mm, besonders bevorzugt etwa 0,1 bis 0,3 mm über den Außenring hervor bzw. heraus.

Bevorzugt ist eine Vielzahl von Signalleitungen bzw. Signalbahnen, beispielsweise zwei, drei, vier, fünf, sechs oder mehr, vorgesehen. Die Signalleitungen bzw. Signalbahnen können grundsätzlich beliebig über den Umfang des Wälzlagers/ Kugellagers/ des Außenrings verteilt sein. Es ist auch denkbar, den gesamten Kreisring des Wälzlagers/ Kugellagers (des Außenrings) auszunutzen. Demnach können die Signalleitungen auch gleichmäßig über den Kreisring verteilt sein.

Eine Obergrenze für die Anzahl von Signalleitungen bzw. Signalbahnen ergibt sich bevorzugt aus der Größe des Wälzlagers/ Kugellagers. Insbesondere hat sich herausgestellt, dass (insbesondere bei dem bevorzugten Bohrungsdurchmesser bzw. Signalleitungsdurchmesser) für das Verhältnis von Außendurchmesser D (in mm) des Kugellagers zur Anzahl der Bohrungen bzw. Signalleitungen N gelten soll: D/N > 0,1. Das Vorsehen einer Vielzahl von Signalleitungen bzw. Signalbahnen um den Umfang verteilt kann in vorteilhafter Weise dazu führen, dass der Übergangswiderstand reduziert wird (Stichwort: parallele Mehrleitertechnik).

Die Offenbarung betrifft weiterhin eine Hülse, welche zur, vorzugsweise multidirektionalen, Weiterleitung bzw. Übertragung von elektrischen Signalen eingerichtet ist und dazu zumindest eine in die Hülse integrierte Signalleitung bzw. Signalbahn aufweist.

Die Hülse ist bevorzugt eine Distanzhülse. Weiter bevorzugt ist die Hülse bzw. Distanzhülse zur Verwendung in einem chirurgischen (Motor-) Instrument, insbesondere einem chirurgischen Handstück, insbesondere einem Fräshandstück, eingerichtet bzw. geeignet bzw. vorgesehen.

Es versteht sich auch hier von selbst, dass die Offenbarung alternativ oder zusätzlich auch eine Verwendung einer Hülse in einem chirurgischen Instrument betrifft.

Bevorzugt ist die Hülse aus einem nichtleitenden Material hergestellt. Weiter bevorzugt handelt es sich bei dem Material der Hülse um ein hartes Material. Als besonders geeignet hat sich dabei Keramik herausgestellt.

Die Signalleitung bzw. Signalbahn ist bevorzugt aus einem (gut) leitenden Material, insbesondere Kupfer, Silber oder Gold, hergestellt.

Die Hülse ist bevorzugt zur Weiterleitung bzw. Übertragung von elektrischen Signalen in einer Axialrichtung zwischen einem ersten axialen Ende und einem zweiten axialen Ende der Hülse und/oder in einer Radialrichtung zwischen einer Innenmantelfläche und einer Außenmantelfläche der Hülse eingerichtet.

In vorteilhafter Weise weist eine Außenmantelfläche der Hülse zumindest eine Rille/ einen Kanal auf, welche/r sich über eine gesamte axiale Länge der Hülse erstreckt. Bevorzugt ist in der Rille/ dem Kanal die Signalbahn bzw. Signalleitung vorgesehen bzw. angeordnet. In anderen Worten befindet sich in der Rille/ dem Kanal leitendes Material. Dadurch wird in vorteilhafter Weise erreicht, dass elektrische Signale an der Außenmantelfläche/ im Außenbereich der Hülse abgegriffen, sowie weitergeleitet bzw. übertragen werden können.

Der zumindest eine Kanal bzw. die zumindest eine Rille ist bevorzugt fein bzw. filigran ausgebildet und durch Schleifen oder Gravieren, insbesondere Lasergravieren, hergestellt. Der Kanal bzw. die Rille wird bevorzugt metallisiert und mit dem gut leitenden Material beschichtet, um die Signalleitung bzw. Signalbahn auszubilden.

Dabei ist es von Vorteil, wenn die Signalbahn bzw. Signalleitung bezüglich der Außenmantelfläche der Hülse nach innen versetzt ist, so dass die Signalbahn bzw. Signalleitung nur in einem unteren Bereich der Rille vorgesehen ist. In anderen Worten ist die Signalbahn bzw. Signalleitung bevorzugt vollständig in der Rille/ dem Kanal versenkt, so dass die (äußere) Außenmantelfläche der Hülse von der Signalbahn bzw. Signalleitung in der Radialrichtung der Hülse beabstandet ist. Somit schließt die Signalbahn/ Signalleitung vorzugsweise nicht bündig mit der Außenmantelfläche ab, sondern befindet sich weiter innen. Insbesondere wenn mehr als eine Signalbahn bzw. Signalleitung vorgesehen ist, wird damit erreicht, dass die einzelnen Signalbahnen bzw. Signalleitungen elektrisch voneinander getrennt sind. Dies ist insbesondere erforderlich, da ein Außenrohr eines Fräshandstücks, in welches die Hülse bevorzugt eingesetzt werden soll und an welchem die Hülse direkt anliegt, häufig aus Metall ist.

Es ist zweckmäßig, wenn über der Signalbahn bzw. Signalleitung ein Isolator angeordnet ist. In anderen Worten kann die angesprochene elektrische Trennung der Signalbahnen bzw. Signalleitungen voneinander verbessert werden, wenn zusätzlich ein Isolator vorgesehen ist. Der Isolator kann beispielsweise ein Einlegeteil sein, insbesondere aus Silikon. Alternativ kann der Isolator beispielsweise auch mittels einer Klebeschicht realisiert werden. Durch die zusätzliche Isolation wird das chirurgische Instrument, insbesondere das Fräshandstück, in welches die Hülse einzusetzen ist, unempfindlicher gegenüber eindringende leitende Flüssigkeiten (z.B. eine Kochsalzlösung).

Ein vorteilhaftes Ausführungsbeispiel ist dadurch gekennzeichnet, dass eine Innenmantelfläche der Hülse zumindest eine Signalbahn bzw. Signalleitung aufweist. Wenn zusätzlich oder alternativ an der Innenfläche der Hülse Signalleitungen bzw. Signalbahnen vorgesehen sind, können in dem Innenbereich elektrische Signale abgegriffen, sowie weitergeleitet bzw. übertragen werden. Beispielsweise können an der Innenmantelfläche metallisierte Bahnen (zumindest eine metallisierte Bahn) vorgesehen sein.

Von besonderem Vorteil ist es, wenn eine an einer Innenmantelfläche der Hülse vorgesehene Signalbahn bzw. Signalleitung elektrisch leitend mit einer an einer Außenmantelfläche der Hülse vorgesehenen Signalbahn bzw. Signalleitung verbunden ist. Beispielsweise kann die Hülse feine Bohrungen (Mikrobohrungen) aufweisen, welche sich in der Radialrichtung der Hülse erstrecken, und über welche eine Signalleitung bzw. Signalbahn an der Innenmantelfläche elektrisch leitend mit einer Signalleitung bzw. Signalbahn an der Außenmantelfläche verbindbar/ verbunden ist (beispielsweise mittels leitendem Material in der Bohrung). In anderen Worten verläuft die Bohrung (Mikrobohrung) bevorzugt zwischen der Rille/ dem Kanal an der Außenmantelfläche und der Signalleitung bzw. Signalbahn an der Innenmantelfläche. Mit anderen Worten werden bevorzugt wie in der Leiterplattentechnik Durchkontaktierungen geschaffen, welche gleichzeitig ebenso als Lötaugen fungieren können. Somit können auch bedrahtete Komponenten in das System integriert werden, sofern bevorzugterweise keine SMD-Komponenten zur Verfügung stehen.

Eine Signalbahn bzw. Signalleitung kann grundsätzlich in unterschiedlicher Tiefe in die Hülse eingebracht sein. Dadurch kann eine zumindest abschnittsweise sehr dünnwandige Hülse realisiert werden. Weiterhin können auch eine Vielzahl von Signalbahnen bzw. Signalleitungen vorgesehen sein, welche in unterschiedlichen Tiefen in die Hülse eingebracht sind. Dies gilt sowohl für an der Außenmantelfläche als auch für an der Innenmantelfläche angebrachte Signalbahnen bzw. Signalleitungen.

Es ist von Vorteil, wenn ein elektrischer Kontakt und/oder eine Ausleseantenne elektrisch leitend mit der Signalleitung bzw. Signalbahn verbunden ist. Dies gilt sowohl für Signalleitungen bzw. -bahnen an der Innenmantelfläche als auch für Signalleitungen bzw. -bahnen an der Außenmantelfläche. Wenn eine Vielzahl von Signalleitungen bzw. -bahnen vorgesehen ist, kann es vorteilhaft sein, wenn eine Signalbahn bzw. -leitung an einer Seite (beispielsweise der Innenseite) unterbrochen ist und an der anderen Seite (beispielsweise der Außenseite) fortgesetzt wird. Dies kann über eine leitende Verbindung in einer radial verlaufenden Bohrung erreicht werden.

Beispielsweise kann an der Innenmantelfläche der Hülse ein elektrischer Kontakt/ eine elektrische Kontaktfläche für einen Sensor oder für ein anderes (elektronisches) Bauteil aufgebracht sein, welche/r bevorzugt elektrisch leitend mit einer an der Innenmantelfläche aufgebrachten Signalleitung bzw. Signalbahn verbunden ist.

Ferner ist es denkbar, dass an der Innenmantelfläche eine Ausleseantenne vorgesehen ist, welche bevorzugt elektrisch leitend mit einer an der Innenmantelfläche aufgebrachten Signalleitung bzw. Signalbahn verbunden ist. Dies kann auch derart realisiert werden, dass die Signalleitung bzw. Signalbahn an der Innenmantelfläche derart angeordnet bzw. ausgebildet ist, dass die Signalleitung bzw. Signalbahn selbst die Ausleseantenne bildet. Eine solche Ausleseantenne kann beispielsweise für das Lesen bzw. Beschreiben eines RFID-Chips verwendet werden.

Außerdem kann auch an der Außenmantelfläche der Hülse ein elektrischer Kontakt/ eine elektrische Kontaktfläche für einen Sensor oder für ein anderes Bauteil aufgebracht sein, welche/r bevorzugt elektrisch leitend mit einer an der Außenmantelfläche aufgebrachten Signalleitung bzw. Signalbahn verbunden ist. Die außen angebrachten elektrischen Kontakte bzw. Kontaktflächen können zur Anbindung von außenliegenden Sensoren, (elektronischen) Bauteilen, (Auslese-) Antennen, etc. genutzt werden.

Außerdem ist es vorteilhaft, wenn die Hülse aus einer Vielzahl von (zumindest zwei, bevorzugt drei oder mehr) ineinander gesetzten Hülsen besteht. In anderen Worten sollen bevorzugt mehrere Hülsen in mehreren Schichten angeordnet werden. Dadurch lassen sich in vorteilhafter Wiese noch mehr Funktionen in die Hülse integrieren und der Bauraum wird maximal ausgenutzt.

Die Offenbarung betrifft weiterhin ein chirurgisches (Motor-) Instrument bzw. Handstück, insbesondere Fräshandstück, mit zumindest einem Wälzlager wie voranstehend beschrieben und zumindest einer Hülse wie voranstehend beschrieben.

Bevorzugt sind in dem chirurgischen Instrument das Wälzlager und die Hülse aneinander angrenzend angeordnet, derart, dass die zumindest eine Signalleitung bzw. Signalbahn des Wälzlagers mit der zumindest einen Signalleitung bzw. Signalbahn der Hülse über eine Steckverbindung verbunden/ konnektiert ist, so dass das chirurgische Instrument zur (multidirektionalen) Signalweiterleitung bzw. Signalübertragung zwischen dem Kugellager und der Hülse eingerichtet ist.

In vorteilhafter Weise können in dem chirurgischen Instrument/ dem Fräshandstück elektrische Signale über das Kugellager und die Hülse bzw. über eine Vielzahl von Kugellagern und eine Vielzahl von Hülsen von einem distalen Bereich zu einem proximalen Bereich des chirurgischen Instruments und umgekehrt, das heißt in Axialrichtung des chirurgischen Instruments, weitergeleitet und übertragen werden.

Dadurch, dass offenbarungsgemäß Wälzlager mit integrierten Signalleitungen und Hülsen mit integrierten Signalleitungen bereitgestellt werden, und die Signalleitungen der Wälzlager mit den Signalleitungen der Hülsen über eine Steckverbindung verbindbar sind, können elektrische Signale sowohl durch diese Bauteile hindurch als auch zwischen diesen Bauteilen übertragen werden.

Das offenbarungsgemäße Wälzlager erlaubt bevorzugt eine Signalübertragung von distal nach proximal und umgekehrt, das heißt in Axialrichtung des chirurgischen Instruments bzw. des Wälzlagers.

Die offenbarungsgemäße Hülse erlaubt bevorzugt eine Signalübertragung sowohl von distal nach proximal und umgekehrt, das heißt in Axialrichtung des chirurgischen Instruments bzw. der Hülse, als auch von innen nach außen und umgekehrt, das heißt in Radialrichtung des chirurgischen Instruments bzw. der Hülse.

Insgesamt wird somit eine multidirektionale Signalweiterleitung/ -übertragung in dem chirurgischen Instrument/ Handstück (Fräshandstück) bereitgestellt, welche durch offenbarungsgemäße Wälzlager und Hülsen mit integrierten Signalleitungen/ - bahnen ermöglicht wird.

Es werden in dem chirurgischen Instrument neue/ erweiterte Funktionen/ Funktionalitäten realisiert, ohne dass der Außendurchmesser bzw. die Außenabmessungen des Schaftes des chirurgischen Handstücks vergrößert wird/ werden. Die offenbarungsgemäße Lösung zeichnet sich durch eine miniaturisierte Signalweiterleitung, eine einfache Montage, erweiterten/ neuen Platzierungsmöglichkeiten von Signalgebern, Antennen bzw. Sensoren, eine Realisierung von komplexen Schaltungen auf kleinstem Bauraum, und eine geeignete Integration in bestehende Bauteile aus.

Die Offenbarung betrifft weiterhin ein Verfahren zur Herstellung eines Wälzlagers/ Kugellagers, insbesondere eines Wälzlagers wie voranstehend beschrieben, mit den Schritten: Bereitstellen zumindest einer in Axialrichtung des Wälzlagers verlaufenden, durchgehenden Bohrung, insbesondere in einem Außenring des Wälzlagers, insbesondere durch Mikrolaserbohren; Einsetzen einer Signalleitung, insbesondere Signallitze, in die Bohrung; und bevorzugt Bereitstellen einer axialen Fixierung der Signalleitung in der Bohrung.

In vorteilhafter Weise wird der Schritt der axialen Fixierung der Signalleitung in der Bohrung bewirkt durch: Plastisches Verformen von axialen Enden der Signalleitung, insbesondere Verstemmen; oder Metallisieren der Bohrung vor dem Einsetzen der Signalleitung in die Bohrung und Bereitstellen einer Klebeverbindung oder Hartlotverbindung nach dem Einsetzen der Signalleitung in die Bohrung.

Ferner betrifft die Offenbarung ein Verfahren zur Herstellung einer Hülse, insbesondere einer Hülse wie voranstehend beschrieben, mit den Schritten: Fertigen der Hülse aus einem nichtleitenden Material; und Bereitstellen einer leitenden Signalbahn bzw. Signalleitung an einer Außenmantelfläche und/oder einer Innenmantelfläche der Hülse durch Metallisieren und gegebenenfalls Beschichten mit einem leitenden Material.

In vorteilhafter Weise weist das Verfahren ferner den Schritt auf: Schleifen oder Gravieren, insbesondere Lasergravieren, von zumindest einem Kanal/ einer Rille in der Außenmantelfläche der Hülse.

Es ist von Vorteil, wenn das Verfahren ferner den Schritt aufweist: Bereitstellen einer in Radialrichtung der Hülse verlaufenden Bohrung zwischen dem Kanal/ der Rille und der Innenmantelfläche der Hülse.

### Kurzbeschreibung der Figuren

Die Offenbarung wird nachfolgend mit Hilfe von Figuren weiter erläutert. Es zeigen:
- Fig. 1A: eine isometrische Ansicht eines offenbarungsgemäßen Kugellagers gemäß einer ersten Ausführungsform;
- Fig. 1B: eine Schnittansicht des offenbarungsgemäßen Kugellagers gemäß der ersten Ausführungsform;
- Fig. 2: eine isometrische Ansicht eines offenbarungsgemäßen Kugellagers gemäß einer zweiten Ausführungsform;
- Fig. 3A: eine erste isometrische Ansicht einer offenbarungsgemäßen Hülse gemäß einer ersten Ausführungsform;
- Fig. 3B: eine zweite isometrische Ansicht der offenbarungsgemäßen Hülse gemäß der ersten Ausführungsform;
- Fig. 3C: eine Schnittansicht der offenbarungsgemäßen Hülse gemäß der ersten Ausführungsform;
- Fig. 3D: eine Detailansicht der offenbarungsgemäßen Hülse gemäß der ersten Ausführungsform;
- Fig. 4A: eine Schnittansicht einer offenbarungsgemäßen Hülse gemäß einer zweiten Ausführungsform;
- Fig. 4B: eine Detailansicht der offenbarungsgemäßen Hülse gemäß der zweiten Ausführungsform;
- Fig. 5A: eine isometrische Ansicht einer offenbarungsgemäßen Hülse gemäß einer dritten Ausführungsform;
- Fig. 5B: eine Schnittansicht der offenbarungsgemäßen Hülse gemäß der dritten Ausführungsform;
- Fig. 6A: eine erste isometrische Ansicht einer offenbarungsgemäßen Hülse gemäß einer vierten Ausführungsform;
- Fig. 6B: eine zweite isometrische Ansicht der offenbarungsgemäßen Hülse gemäß der vierten Ausführungsform;
- Fig. 6C: eine Schnittansicht der offenbarungsgemäßen Hülse gemäß der vierten Ausführungsform;
- Fig. 7A: eine erste isometrische Ansicht einer offenbarungsgemäßen Hülse gemäß einer fünften Ausführungsform;
- Fig. 7B: eine zweite isometrische Ansicht der offenbarungsgemäßen Hülse gemäß der fünften Ausführungsform;
- Fig. 8A: eine isometrische Ansicht einer offenbarungsgemäßen Hülse in Multilayer-Bauart in einem montierten Zustand gemäß einer sechsten Ausführungsform;
- Fig. 8B: eine isometrische Ansicht der Hülse von Fig. 8A in halbtransparenter Darstellung;
- Fig. 8C: eine erste isometrische Ansicht einer äußeren Hülse der Multilayer-Hülse von Fig. 8A;
- Fig. 8D: eine zweite isometrische Ansicht der äußeren Hülse der Multilayer-Hülse von Fig. 8A;
- Fig. 8E: eine erste isometrische Ansicht einer mittleren Hülse der Multilayer-Hülse von Fig. 8A;
- Fig. 8F: eine zweite isometrische Ansicht der mittleren Hülse der Multilayer-Hülse von Fig. 8A;
- Fig. 8G: eine erste isometrische Ansicht einer inneren Hülse der Multilayer-Hülse von Fig. 8A;
- Fig. 8H: eine zweite isometrische Ansicht der inneren Hülse der Multilayer-Hülse von Fig. 8A;
- Fig. 9A: eine Schnittansicht des offenbarungsgemäßen chirurgischen Instruments;
- Fig. 9B: eine Schnittansicht eines vorderen distalen Bereichs des chirurgischen Instruments von Fig. 9A;
- Fig. 9C: eine Schnittansicht eines mittleren distalen Bereichs des chirurgischen Instruments von Fig. 9A; und
- Fig. 9D: eine isometrische Ansicht einer Anordnung von Kugellagern und Hülsen in dem offenbarungsgemäßen chirurgischen Instrument.

### Beschreibung der Ausführungsformen/ -beispiele

Nachstehend werden Ausführungsformen der vorliegenden Offenbarung auf der Grundlage der zugehörigen Figuren beschrieben.

Die Figuren sind lediglich schematischer Natur und dienen ausschließlich dem Verständnis der Offenbarung. Gleiche Elemente sind mit denselben Bezugszeichen versehen. Die Merkmale der einzelnen Ausführungsbeispiele/ -formen können untereinander ausgetauscht werden.

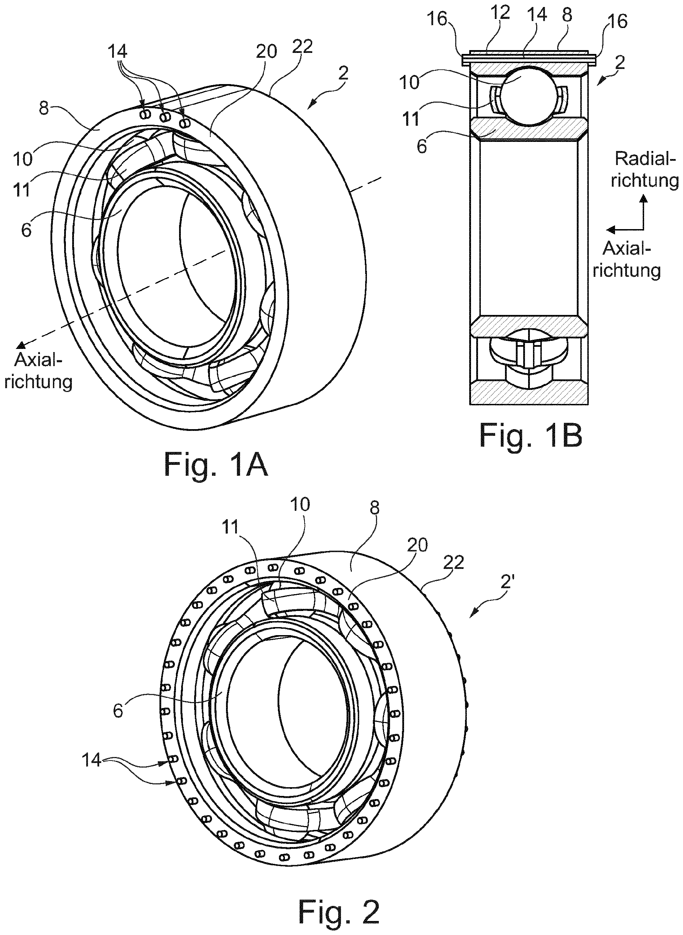

Fig. 1A und Fig. 1B zeigen eine erste Ausführungsform eines offenbarungsgemäßen Kugellagers 2. Das Kugellager 2 ist ein Mikro-Kugellager und vorgesehen, in einem chirurgischen Instrument 4 (nicht gezeigt in Fig. 1A und Fig. 1B) verwendet zu werden. Das Kugellager 2 weist einen Innenring 6 und einen Außenring 8 auf. Zwischen dem Innenring 6 und dem Außenring 8 sind rollende (Wälz-) Körper, insbesondere Kugeln 10, vorgesehen, welche von einem Käfig 11 gehalten sind. Es sei an dieser Stelle noch einmal angeführt, dass die Offenbarung grundsätzlich nicht auf ein Kugellager 2 beschränkt ist, sondern auch beliebige andere Wälzlager wie etwa Zylinderrollenlager, Nadellager, Kegelrollenlager, Tonnenlager, Toroidalrollenlager, mit oder ohne Kugelkäfig, etc. von der Offenbarung umfasst sein sollen.

Der Außenring 8 des Kugellagers ist vorliegend aus einem nichtleitenden, harten Material, beispielsweise Keramik hergestellt. In dem Außenring 8 des Kugellagers 2 sind drei sich in Axialrichtung des Kugellagers 2 erstreckende Bohrungen 12 vorgesehen. Wie insbesondere aus Fig. 1B hervorgeht, erstrecken sich die Bohrungen 12 über eine gesamte axiale Länge des Kugellagers 2 und sind demnach Durchgangsbohrungen. Ein Durchmesser der Bohrungen ist bevorzugt kleiner als 0,2 mm, weiter bevorzugt etwa im Bereich von 0,1 mm. Die Bohrungen werden beispielsweise durch Mikrolaserbohren hergestellt. In die Bohrungen 12 sind Signalleitungen (Signallitzen) 14 eingesetzt, welches sich über die gesamte axiale Länge der Bohrungen 12 erstrecken. Die Signalleitungen 14 sind vorliegend aus einem gut leitenden Material, insbesondere Kupfer, Silber oder Gold, hergestellt. Ein Durchmesser der Signalleitungen 14 entspricht etwa einem Durchmesser der Bohrungen und ist daher auch kleiner als 0,2 mm, vorzugsweise im Bereich von 0,1 mm.

Die Signalleitungen 14 sind in den Bohrungen 12 axial fixiert. Diese axiale Fixierung wird vorliegend realisiert, indem axiale Enden 16 der Signalleitungen 14 plastisch verformt, insbesondere verstemmt werden. Dann weisen die Signalleitungen 14 an ihren axialen Enden 16 einen leicht vergrößerten Durchmesser auf, wie aus Fig. 1B hervorgeht. Alternativ können die Bohrungen 12 auch zunächst metallisiert werden (bevor die Signalleitungen 14 eingesetzt werden) und die axiale Fixierung kann über eine Klebeverbindung oder eine Lotverbindung erfolgen.

Die (beiden) axialen Enden 16 der Signalleitungen 14 stehen in Axialrichtung des Kugellagers 2 über den Außenring 8 hervor/ heraus, bevorzugt um etwa 0,1 bis 0,3 mm. Dies dient dazu, dass die Signalleitungen 14 mit einem anderen Bauteil des chirurgischen Instrument 4, beispielsweise einer Hülse 18, eine Steckverbindung eingehen können.

Somit wird gemäß der in Fig. 1A und Fig. 1B gezeigten Ausführungsform ein Kugellager 2 mit in das Kugellager 2 integrierten Signalleitungen 14 bereitgestellt. Das Kugellager kann über die Signalleitungen 14 elektrische Signale von einem ersten axialen Ende 20 zu einem zweiten axialen Ende 22 des Kugellagers 2 (von proximal nach distal und umgekehrt, bzw. in Axialrichtung des Kugellagers 2) weiterleiten bzw. übertragen.

Zwar sind in der ersten Ausführungsform des offenbarungsgemäßen Kugellagers 2 drei Bohrungen 12 und somit drei Signalleitungen 14 vorgesehen, jedoch ist die Offenbarung nicht darauf begrenzt und es können auch eins, zwei, vier, fünf, sechs oder mehr Signalleitungen 14 vorgesehen sein, welche beliebig über den Umfang des Außenrings 8 des Kugellagers 2 verteilt sein können.

Gemäß einer zweiten Ausführungsform des offenbarungsgemäßen Kugellagers 2' wird der gesamte Außenring 8 des Kugellagers 2' ausgenutzt und mit Bohrungen 12 und Signalleitungen 14 versehen (siehe Fig. 2). Die Bohrungen 12 bzw. Signalleitungen 14 sind hier gleichmäßig über den Außenring 8 des Kugellagers 2' verteilt. Das in Fig. 2 gezeigte Kugellager 2' weist beispielsweis N = 36 Bohrungen 12 bzw. Signalleitungen 14 auf und hat einen Außendurchmesser von 4,763 mm, so dass das angesprochene Verhältnis von D/N > 0,1 (noch) erfüllt ist. Der Durchmesser der Signalleitungen 14 ist in der in Fig. 2 gezeigten Ausführungsform bevorzugt näherungsweise/ gerundet 0,1 mm.

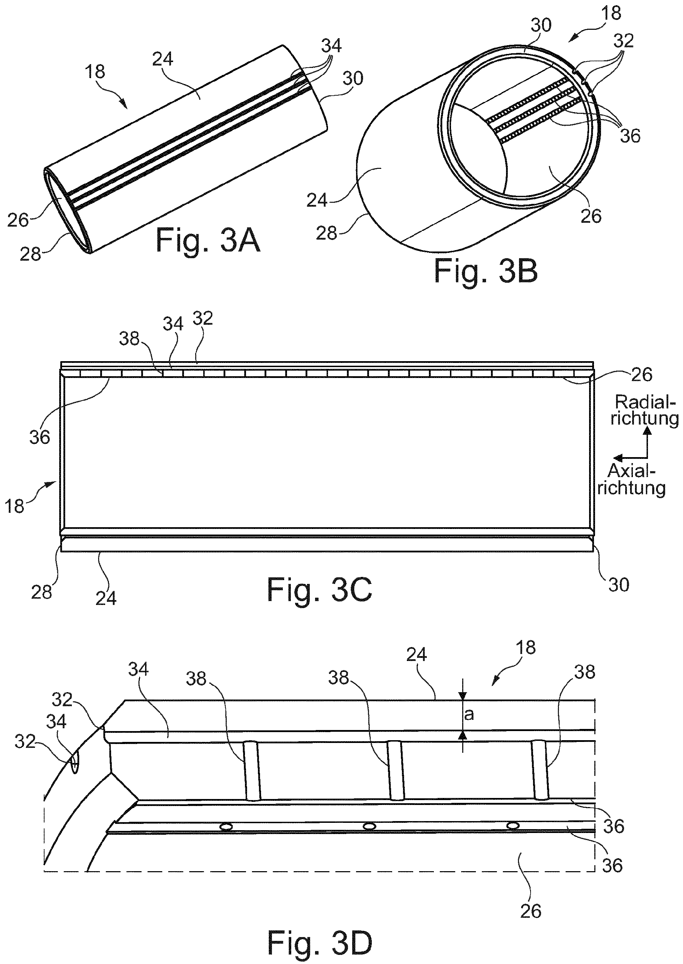

Fig. 3A, Fig. 3B, Fig. 3C und Fig. 3D zeigen eine Hülse 18 gemäß einer ersten offenbarungsgemäßen Ausführungsform. Die Hülse 18 ist bevorzugt eine Distanzhülse und ist vorgesehen, in einem chirurgischen Instrument 4 (nicht gezeigt in Fig. 3A bis Fig. 3D) verwendet zu werden. Die Hülse 18 ist aus einem nichtleitenden, harten Material, beispielsweise Keramik hergestellt. Die Hülse 18 weist grundsätzlich eine Außenmantelfläche 24, eine Innenmantelfläche 26, ein erstes axiales Ende 28 und ein zweites axiales Ende 30 auf.

An der Außenmantelfläche 24 der Hülse 18 sind vorliegend drei gerade/ geradlinige Rillen (Kanäle) 32 vorgesehen, welche sich über eine gesamte axiale Länge der Hülse 18 erstrecken. Die Rillen 32 sind relativ fein bzw. filigran ausgebildet und werden durch Schleifen oder Gravieren, insbesondere Lasergravieren, hergestellt. In den Rillen 32 sind Signalleitungen 34 vorgesehen, welche sich ebenso über die gesamte axiale Länge der Hülse 18 erstrecken. Die Signalleitungen (Signalbahnen) 34 werden ausgebildet, indem die Rillen 32 zunächst metallisiert werden und anschließend mit einem gut leitenden Material, insbesondere Kupfer, Silber oder Gold, beschichtet werden.

Die Signalleitungen 34 an der Außenmantelfläche 24 der Hülse 18 sind nur in einem unteren Bereich der Rille 32 vorgesehen, wie insbesondere aus Fig. 3D hervorgeht, so dass die äußere Außenmantelfläche 24 der Hülse 18 von jeder Signalleitung (Signalbahn) 34 in der Radialrichtung der Hülse 18 beabstandet ist, insbesondere einen Abstand a aufweist.

Zwar sind in der ersten Ausführungsform der offenbarungsgemäßen Hülse 18 drei Signalleitungen 34 an der Außenmantelfläche 24 vorgesehen, jedoch ist die Offenbarung nicht darauf begrenzt und es können auch eins, zwei, vier, fünf, sechs oder mehr Signalleitungen 34 vorgesehen sein, welche beliebig über die Außenmantelfläche 24 der Hülse 18 verteilt sein können. Es ist auch möglich, die gesamte Außenmantelfläche 24 der Hülse 18 auszunutzen, und somit Signalleitungen 34 (gleichmäßig) über die gesamte Außenmantelfläche 24 zu verteilen.

An der Innenmantelfläche 26 der Hülse 18 sind drei gerade/ geradlinige Signalleitungen (Signalbahnen) 36 vorgesehen. Die Signalleitungen 36 an der Innenmantelfläche 26 erstrecken sich über eine gesamte axiale Länge der Hülse 18 und sind vorliegend als metallisierte (Signal-) Bahnen ausgebildet.

Jede Signalleitung 36 an der Innenmantelfläche 26 der Hülse 18 ist vorliegend in der Umfangsrichtung der Hülse 18 an derselben Stelle wie eine Signalleitung 34 an der Außenmantelfläche 24 der Hülse 18 vorgesehen. Somit verlaufen die Signalleitungen 34 und die Signalleitungen 36 parallel und geradlinig in der Axialrichtung der Hülse 18 und sind in der Umfangsrichtung der Hülse 18 an derselben Position vorgesehen.

Bevorzugt ist zumindest eine Signalleitung 36 an der Innenmantelfläche 26 der Hülse 18 elektrisch leitend mit einer Signalleitung 34 an der Außenmantelfläche 24 der Hülse 18 verbunden. Es kann grundsätzlich auch jede Signalleitung 36 mit einer entsprechenden Signalleitung 34 elektrisch leitend verbunden sein. Vorliegend sind in der Hülse 18 feine (Mikro-) Bohrungen 38 zwischen der Rille 32/ der Signalleitung 34 an der Außenmantelfläche 24 und der Signalleitung 36 an der Innenmantelfläche 26 vorgesehen, welche sich jeweils in einer Radialrichtung der Hülse 18 erstrecken. Die Bohrungen 38 enthalten (gut) leitendes Material, so dass die Signalleitungen 34 über das leitende Material in den Bohrungen 38 elektrisch leitend mit den Signalleitungen 36 verbunden sind.

Zwar sind in der ersten Ausführungsform der offenbarungsgemäßen Hülse 18 drei Signalleitungen 36 an der Innenmantelfläche 26 vorgesehen, jedoch ist die Offenbarung nicht darauf begrenzt und es können auch eins, zwei, vier, fünf, sechs oder mehr Signalleitungen 36 vorgesehen sein, welche beliebig über die Innenmantelfläche 26 der Hülse 18 verteilt sein können. Es ist auch möglich, die gesamte Innenmantelfläche 26 der Hülse 18 auszunutzen, und somit Signalleitungen 36 (gleichmäßig) über die gesamt Innenmantelfläche 26 zu verteilen.

Insgesamt ist die Hülse 18 gemäß der ersten Ausführungsform eine Hülse 18, welche integrierte Signalleitungen 34, 36 aufweist. Mit den Signalleitungen 34 an der Außenmantelfläche 24 der Hülse 18 können in einem Außenbereich der Hülse 18 elektrische Signale abgegriffen, weitergeleitet und übertragen werden. Mit den Signalleitungen 36 an der Innenmantelfläche 26 der Hülse 18 können in einem Innenbereich der Hülse 18 elektrische Signale abgegriffen, weitergeleitet und übertragen werden. Die offenbarungsgemäße Hülse 18 ermöglicht grundsätzlich eine Weiterleitung bzw. Übertragung von elektrischen Signalen sowohl in der Axialrichtung zwischen dem ersten axialen Ende 28 der Hülse 18 und dem zweiten axialen Ende 30 der Hülse 18 als auch in der Radialrichtung der Hülse 18 zwischen der Innenmantelfläche 26 und der Außenmantelfläche 24. Somit ist die Hülse 18 zur multidirektionalen Weiterleitung bzw. Übertragung von elektrischen Signalen eingerichtet.

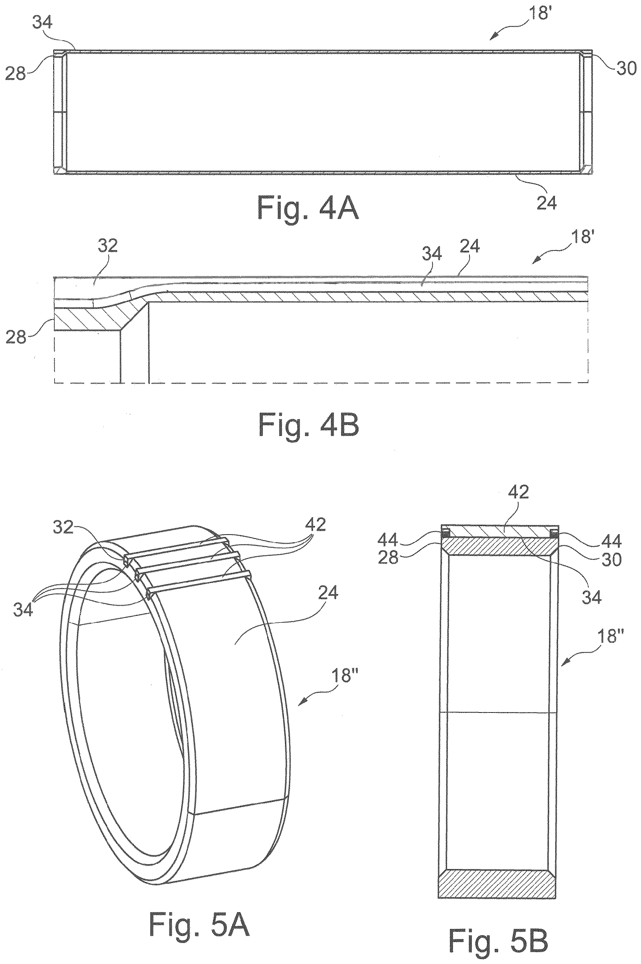

Fig. 4A und Fig. 4B zeigen eine offenbarungsgemäße Hülse 18' gemäß einer zweiten Ausführungsform. Die Hülse 18' gemäß der zweiten Ausführungsform weist eine Signalleitung/ Signalbahn 34 an der Außenmantelfläche 24 auf. Grundsätzlich ist die Hülse 18' sehr dünnwandig. Lediglich an dem ersten axialen Ende 28 der Hülse 18' und an dem zweiten axialen Ende 30 der Hülse 18' ist die Hülse 18' etwas dickwandiger. Die an der Außenmantelfläche 24 vorgesehene Rille 32 weist unterschiedliche Tiefen auf. Insbesondere ist eine Tiefe der Rille 32 an dem ersten axialen Ende 28 und dem zweiten axialen Ende 30 der Hülse 18' tiefer/ größer als in dem restlichen, verbleibenden (mittleren) Bereich der Hülse 18'. Wie insbesondere aus Fig. 4B hervorgeht, ist demnach auch die Signalleitung/ Signalbahn 34 in unterschiedlicher Tiefe in die Hülse 18' eingebracht (tiefer an den axialen Enden 28, 30), so dass insbesondere in dem mittleren Bereich der Hülse 18' eine äußerst dünnwandige Hülse 18' realisiert wird.

Es sei an dieser Stelle angeführt, dass grundsätzlich auch eine Signalleitung 34, 36 der Hülse 18 gemäß der ersten offenbarungsgemäßen Ausführungsform (siehe Fig. 3A bis 3D) in unterschiedlichen Tiefen in die Hülse 18 eingebracht sein kann (entsprechend der zweiten Ausführungsform). Dies gilt sowohl für an der Außenmantelfläche 24 als auch für an der Innenmantelfläche 26 angebrachte Signalleitungen 34, 36. Auch können die Rillen 32 insgesamt unterschiedliche Tiefen aufweisen, das heißt eine erste Rille 32 kann tiefer als eine zweite Rille 32 sein, so dass entsprechend auch eine erste Signalleitung 34 tiefer als eine zweite Signalleitung 34 in die Hülse 18 eingebracht sein kann/ in der Axialrichtung der Hülse 18 verlaufen kann.

Fig. 5A und Fig. 5B zeigen eine offenbarungsgemäße Hülse 18" gemäß einer dritten Ausführungsform. Die Hülse 18" gemäß der dritten Ausführungsform weist drei Signalleitungen/ Signalbahnen 34 an der Außenmantelfläche 24 auf. Bevor die Hülse 18" der dritten Ausführungsform weiter beschrieben wird, werden die Ausführungen zur Hülse 18 der ersten Ausführungsform (sieh Fig. 3A bis Fig. 3D) noch ergänzt: Der Grund, warum die Signalleitungen 34 an der Außenmantelfläche 24 der Hülse 18 nur in einem unteren Bereich der Rille 32 vorgesehen sind, ist insbesondere der, dass dadurch eine elektrische Trennung der Signalleitungen 34 voneinander gewährleistet wird. Dies ist erforderlich, da ein Außenrohr 40 eines chirurgischen Instruments 4, in welches die Hülse 18 bevorzugt eingesetzt werden soll und an welchem die Hülse 18 direkt anliegt, häufig aus Metall ist.

Mit der Hülse 18" gemäß der dritten Ausführungsform wird erreicht, dass die angesprochene elektrische Trennung der Signalleitungen 34 noch weiter verbessert wird. Dazu ist in der Hülse 18" auf jeder Signalleitung 34 ein Isolator 42 angeordnet. Der Isolator 42 ist vorliegend ein Einlegeteil, insbesondere aus Silikon. Alternativ kann der Isolator 42 auch mittels einer Klebeschicht realisiert werden. Der Isolator 42 ist in der Rille 32 angeordnet wird in die Rille 32 eingesetzt und befindet sich auf der Signalleitung 34 bzw. in anderen Worten radial weiter außen im Hinblick auf die Signalleitung 34. Der Isolator 42 erstreckt sich über eine gesamte axiale Länge der Rille 32 bzw. der Signalleitung 34 und bedeckt somit die Signalleitung 34 vollständig.

Wie insbesondere aus Fig. 5B hervorgeht, sind an dem ersten axialen Ende 28 der Hülse 18" und an dem zweiten axialen Ende 30 der Hülse 18" in dem Bereich, in welchem der Isolator 42 auf die Signalleitung 34 aufgebracht ist, Aussparungen 44 vorgesehen. Die Aussparungen 44 sind insbesondere vorgesehen, um eine Steckverbindung mit den vorstehenden axialen Enden 16 des Kugellagers 2, 2' (den Signalkontakten des Kugellagers 2, 2') zu ermöglichen.

Durch die zusätzliche Isolation, welche von dem Isolator 42 bereitgestellt wird, wird das chirurgische Instrument (Fräshandstück) 4, in welches die Hülse 18" einzusetzen ist, unempfindlicher gegenüber eindringende leitende Flüssigkeiten (z.B. eine Kochsalzlösung).

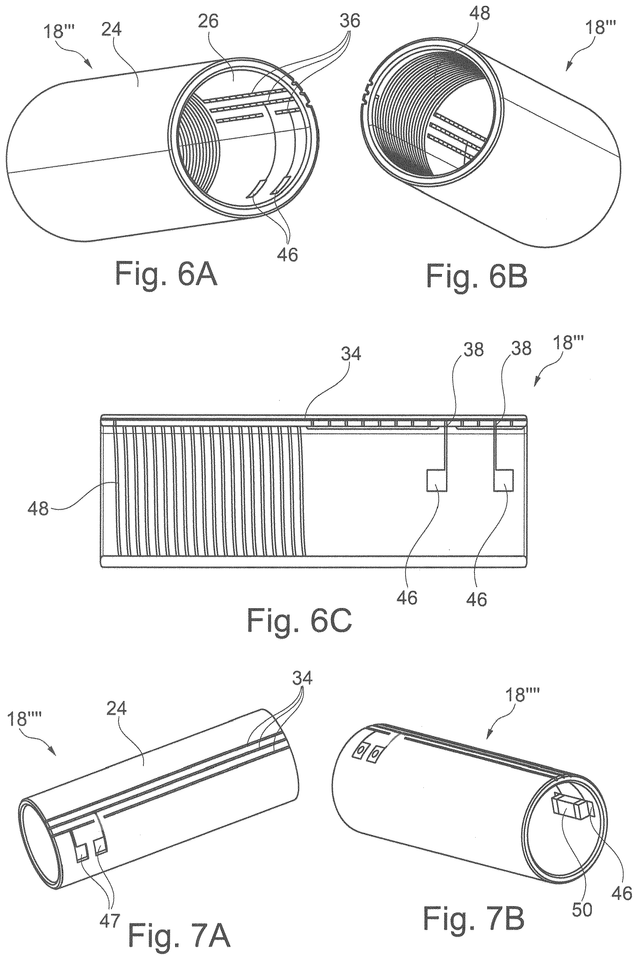

Fig. 6A, Fig. 6B und Fig. 6C zeigen eine offenbarungsgemäße Hülse 18‴ gemäß einer vierten Ausführungsform. Wie insbesondere aus Fig. 6A hervorgeht, sind an der Innenmantelfläche 26 der Hülse 18'" elektrische Kontakte/ Kontaktflächen 46 vorgesehen. Die elektrischen Kontakte/ Kontaktflächen 46 dienen grundsätzlich als Kontakte/ Kontaktflächen für Sensoren oder andere (elektronische) Bauteile. Jede/r der elektrischen Kontakte/ Kontaktflächen 46 ist elektrisch leitend mit jeweils einer Signalleitung 36 verbunden, das heißt ein erster elektrischer Kontakt 46 der zwei elektrischen Kontakte 46 ist elektrisch leitend mit einer ersten Signalleitung 36 der drei Signalleitungen 36 verbunden und ein zweiter elektrischer Kontakt 46 der zwei elektrischen Kontakte 46 ist elektrisch leitend mit einer zweiten Signalleitung 36 der drei Signalleitungen 36 verbunden. Wie aus Fig. 6A hervorgeht, sind vorliegend die Signalleitungen 36, welche mit elektrischen Kontakten 46 verbunden sind, an der Innenmantelfläche 26 der Hülse 18‴ unterbrochen. Diese hier unterbrochenen Signalleitungen 36 sind mittels leitendem Material in den (Mikro-) Bohrungen 38 (vgl. erste Ausführungsform der Hülse 18) elektrisch leitend mit Signalleitungen 34 an der Außenmantelfläche 24 verbunden und werden entsprechend an der Außenseite der Hülse 18‴ fortgesetzt.

Darüber hinaus ist eine an der Innenmantelfläche 26 vorgesehene Signalleitung 36 (die dritte Signalleitung 36) elektrisch leitend mit einer Ausleseantenne 48 verbunden. Wie insbesondere aus Fig. 6B und Fig. 6C hervorgeht, verhält es sich vorliegend sogar so, dass die dritte Signalleitung 36 an der Innenmantelfläche 26 derart ausgebildet ist, dass die dritte Signalleitung 36 selbst die Ausleseantenne 48 bildet. Dies wird dadurch erreicht, dass die dritte Signalleitung 36 abschnittsweise nicht in der Axialrichtung der Hülse 18‴ verläuft, sondern spiralförmig/ spulenförmig, also Windungen aufweisend ausgebildet ist, und somit näherungsweise in der Umfangsrichtung der Hülse 18‴ verläuft (vgl. Fig. 6C). Die Ausleseantenne 48 wird bevorzugt für das Lesen bzw. Beschreiben eines (nicht gezeigten) RFID-Chips verwendet. Der RFID-Chip kann zum Beispiel in einem (nicht gezeigten) Werkzeug vorgesehen sein, welches in das chirurgische Instrument (Fräshandstück) eingesteckt wird, z.B. ein Fräswerkzeug.

Im Übrigen sind im Hinblick auf die Hülse 18‴ der vierten Ausführungsform die Beschreibungen der Hülse 18, 18', 18" der vorherigen Ausführungsformen, insbesondere die Beschreibung der Hülse 18 der ersten Ausführungsform, anwendbar.

Fig. 7A und Fig. 7B zeigen eine offenbarungsgemäße Hülse 18ʺʺ gemäß einer fünften Ausführungsform. In der Hülse 18ʺʺ sind an der Außenmantelfläche 24 zwei elektrische Kontakte/ Kontaktflächen 47 vorgesehen, welche elektrisch leitend mit jeweils einer Signalleitung 34 verbunden sind. Es sind somit offenbarungsgemäß neben oder alternativ zu elektrischen Kontakten/ Kontaktflächen 46, welche an der Innenmantelfläche 26 (innen) vorgesehen sind (vgl. vierte Ausführungsform), auch elektrische Kontakte/ Kontaktflächen 47 denkbar, welche an der Außenmantelfläche 24 (außen) vorgesehen sind.

Die außen vorgesehenen elektrischen Kontakte/ Kontaktflächen 47 können zur Anbindung von außenliegenden (elektronischen) Bauteilen, etwa Sensoren oder Antennen, verwendet werden. Hier wäre es erforderlich, in dem Außenrohr 40 des chirurgischen Instruments (Fräshandstücks) 4 eine (nicht gezeigte) Ausnehmung vorzusehen, welche den Bauraum für das außenliegende (elektronische) Bauteil (z.B. Sensor oder Antenne) bildet. Es ist auch denkbar, dass über die elektrischen Kontakte/ Kontaktflächen 47 an der Außenmantelfläche 24 elektrische Signale nach außen (z.B. an das Außenrohr 40 des chirurgischen Instruments 4) weitergeleitet werden. Dies kann beispielsweise in einem proximalen Bereich des chirurgischen Instruments 4 sinnvoll sein, in welchem sich der Bauraum des chirurgischen Instruments 4 vergrößert.

Auch in dieser Ausführungsform können Signalleitungen 34, 36, welche an einer Seite (zum Beispiel eine Signalleitung 34 außen in Fig. 7A) unterbrochen sind, durch jeweils durchgehende Signalleitungen 34, 36 an der Gegenseite (zum Beispiel eine Signalleitung 36 innen) fortgesetzt werden. Auch ist in Fig. 7B erkennbar, dass gemäß der vorliegenden fünften Ausführungsform bevorzugt auch innen/ an der Innenmantelfläche 26 ein elektrischer Kontakt 46/ elektrische Kontakte 46 vorgesehen ist/ sind, an welchem/n ein Sensor 50 angebracht ist.

Im Übrigen sind im Hinblick auf die Hülse 18ʺʺ der fünften Ausführungsform die Beschreibungen der Hülse 18, 18', 18", 18‴ der vorherigen Ausführungsformen, insbesondere die Beschreibungen der Hülse 18, 18‴ der ersten und vierten Ausführungsformen, anwendbar.

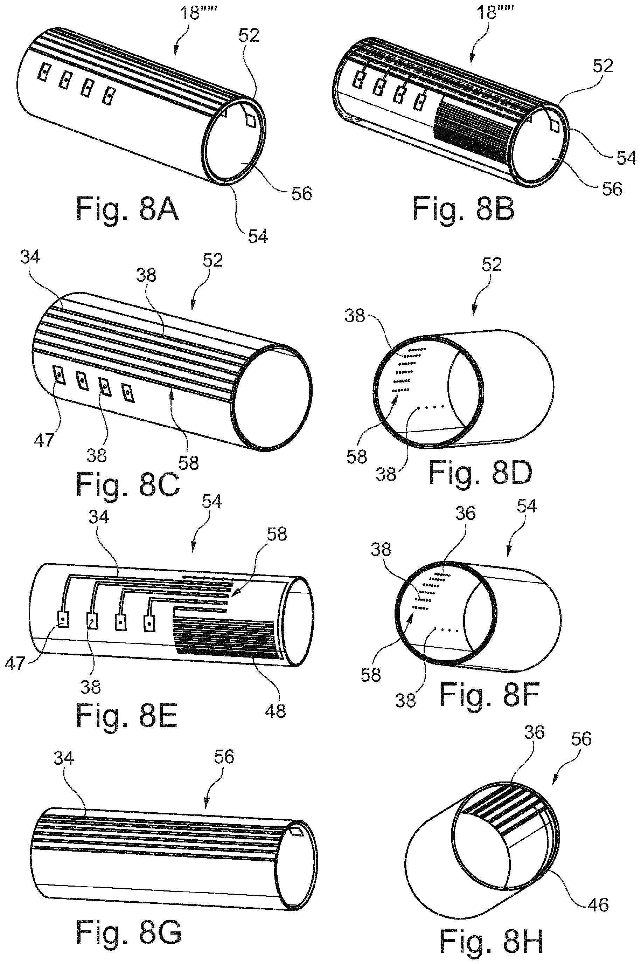

Fig. 8A und Fig. 8B zeigen eine offenbarungsgemäße Hülse 18‴ʺ gemäß einer sechsten Ausführungsform der vorliegenden Offenbarung. Die Hülse 18‴ʺ ist grundsätzlich eine Multilayer-Hülse und besteht aus einer Vielzahl von ineinander gesetzten, das heißt in mehreren Schichten angeordneten, Hülsen bzw. Rohren. Vorliegend sind insbesondere drei Hülsen/ Rohre ineinander gesetzt, nämlich eine äußere Hülse 52, welche in Fig. 8C und Fig. 8D dargestellt ist, eine mittlere Hülse 54, welche in Fig. 8E und 8F dargestellt ist, und eine innere Hülse 56, welche in Fig. 8G und 8H dargestellt ist.

Die äußere Hülse 52 weist außen (an ihrer Außenmantelfläche) Signalleitungen/ Signalbahnen 34 und elektrische Kontakte/ Kontaktflächen 47 auf. Insbesondere sind vorliegend sechs Signalleitungen 34 und vier elektrische Kontakte 47 vorgesehen. Über Mikrobohrungen 38, welche leitendes Material enthalten können, sind sowohl die Signalleitungen 34 als auch die elektrischen Kontakte 47 nach innen (zu der Innenmantelfläche der Hülse 52) durchverbunden/ durchverbindbar. Die Mikrobohrungen 38, welche die Signalleitungen 34 nach innen durchverbinden können, sind vorliegend nur in einem definierten (bestimmten) Verbindungsbereich 58 vorgesehen.

Die mittlere Hülse 54 weist außen (an ihrer Außenmantelfläche) auch Signalleitungen/ Signalbahnen 34 und elektrische Kontakte/ Kontaktflächen 47 auf. Darüber hinaus ist außen auch eine Ausleseantenne 48 vorgesehen. Zwei Signalleitungen 34 der vorgesehenen sechs Signalleitungen 34 sind mit der Ausleseantenne 48 verbunden. Die verbleibenden vier Signalleitungen sind mit jeweils einem elektrischen Kontakt 47 verbunden. Über Mikrobohrungen 38, welche leitendes Material enthalten können, sind auch in der mittleren Hülse 54 die Signalleitungen 34 und die elektrischen Kontakte 47 nach innen (zu der Innenmantelfläche der Hülse 54) durchverbunden/ durchverbindbar. Die Mikrobohrungen, welche die Signalleitungen 34 nach innen durchverbinden, sind nur in einem definierten (bestimmten) Verbindungsbereich 58 vorgesehen und verbinden die außenliegenden Signalleitungen 34 mit innen liegenden Signalleitungen 36. Sowohl die außenliegenden Signalleitungen 34 als auch die innenliegenden Signalleitungen 36 erstrecken sich an der mittleren Hülse 54 nicht über die gesamte axiale Länge der Hülse 54.

Die mittlere Hülse 54 wird insbesondere genutzt für eine Verbindung/ Konnektierung/ Verschaltung (von Signalleitungen oder elektrischen Kontakten) von innen nach außen oder von außen nach innen (in der Radialrichtung der Hülse 18‴ʺ). Weiterhin können über die mittlere Hülse 54 zusätzliche Signalleitungen/ Signalbahnen (leitende Bahnen) aufgebracht werden (beispielsweise in Antennenform, um die Ausleseantenne 48 zu bilden).

Die innere Hülse 56 weist außen (an ihrer Außenmantelfläche) Signalleitungen/ Signalbahnen 34 auf. Innen (an ihrer Innenmantelfläche) weist sie Signalleitungen/ Signalbahnen 36 auf. Die außenliegenden Signalleitungen 34 erstrecken sich über die gesamte axiale Länge der inneren Hülse 56 und sind daher durchgängig. Die innere Hülse 56 weist innen zwei elektrische Kontakte 46 auf, welche mit jeweils einer Signalleitung 36 verbunden sind. Um die Verbindung der elektrischen Kontakte 46 mit den Signalleitungen 36 zu realisieren, ist vorliegend eine Signalleitung 36 unterbrochen. Grundsätzlich ist dann eine Durchschaltung von der inneren Hülse 56 an eine Signalleitung 34 (außen) der inneren Hülse 56 denkbar. Es kann jedoch auch eine Durchschaltung direkt weiter auf die mittlere Hülse 54 erfolgen. Von der mittleren Hülse 54 kann schließlich eine Durchschaltung sowohl an die äußere Hülse 52 als auch zurück an die innere Hülse 56, insbesondere eine andere Signalleitung 34, 36 der inneren Hülse 56, erfolgen.

Wenn nur ein elektrischer Kontakt 46 oder kein elektrischer Kontakt 46 vorgesehen ist, können auch alle Signalleitungen 36 durchgängig sein, das heißt sich über die gesamte axiale Länge der inneren Hülse 52 erstrecken.

Wie insbesondere aus der halbtransparenten Darstellung von Fig. 8B hervorgeht, sind die Signalleitungen 34, 36 der Hülsen 52, 54, 56 in Deckung, also direkt über- bzw. untereinander angeordnet, wenn die Multilayer-Hülse 18‴ʺ montiert ist. Auch die an der äußeren Hülse 52 und der mittleren Hülse 54 vorgesehenen Verbindungsbereiche 58 sind in Deckung, also direkt über- bzw. untereinander angeordnet. Dasselbe gilt für die an sowohl der äußeren Hülse 52 als auch an der mittleren Hülse 54 angeordneten Kontaktflächen 47.

Eine elektrische Verbindung zwischen den drei Hülsen 52, 54, 56 wird über die Mikrobohrungen 38 realisiert. Insbesondere kann eine Konnektierung an bestimmten, definierten Punkten mittels Lot oder anderer metallischer Verbindung erfolgen. In die Hülse 18‴ʺ gemäß der sechsten Ausführungsform können im Vergleich zu den vorher beschriebenen Ausführungsformen noch mehr Funktionen integriert werden und der vorhandene Bauraum wird maximal ausgenutzt.

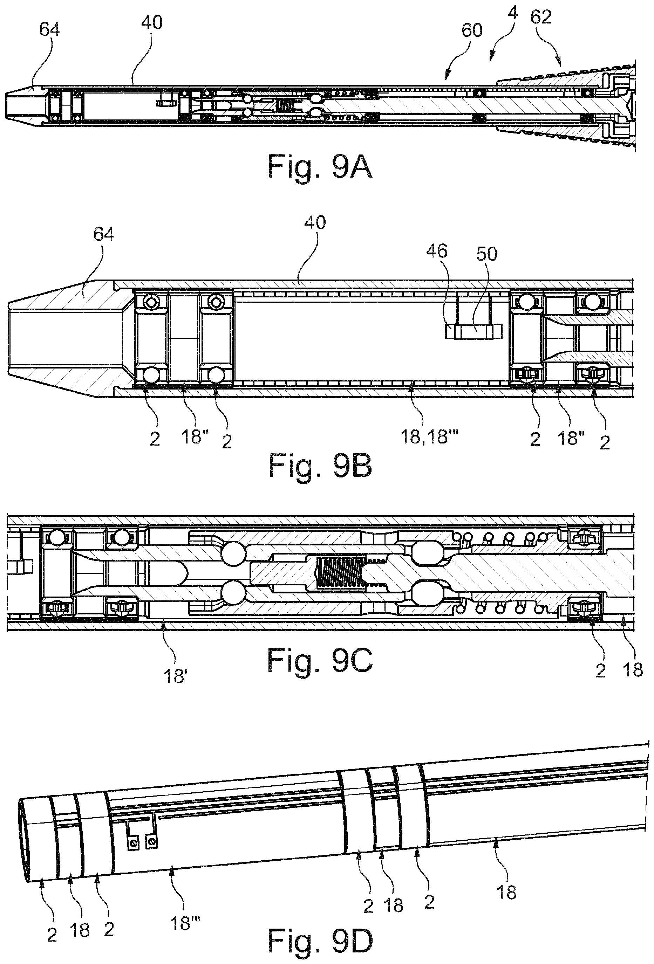

Fig. 9A zeigt eine Schnittansicht eines offenbarungsgemäßen chirurgischen Instruments 4. Das chirurgische Instrument 4 ist vorliegend bevorzugt ein chirurgisches Handstück, weiter bevorzugt ein Fräshandstück. Das chirurgische Instrument 4 weist grundsätzlich einen distalen Bereich 60 und einen proximalen Bereich 62 auf. Der proximale Bereich 62 zeichnet sich dadurch aus, dass sich in diesem der Bauraum vergrößert. In dem distalen Bereich 60 ist grundsätzlich das Außenrohr 40 (ein zylindrisches, längliches Rohr) des chirurgischen Instruments (Fräshandstücks) 4 vorgesehen. An dem distalen Ende des Außenrohrs 40 ist ein Koppelelement 64 angeordnet, welches einer Aufnahme eines Werkzeugs, beispielsweise eines Fräswerkzeugs, dient.

Gemäß der vorliegenden Offenbarung sollen elektrische Signale von einem distalen Ende des chirurgischen Instruments 4 bis in den proximalen Bereich 62 übertragen werden, ohne dass sich der Bauraum vergrößert, das heißt ohne dass beispielsweise der Außendurchmesser des Außenrohrs 40 größer wird. Dies wird offenbarungsgemäß erreicht, wenn innerhalb des Außenrohrs 40 die offenbarungsgemäßen Kugellager 2, 2' und die offenbarungsgemäßen Hülsen 18, 18', 18", 18‴, 18"", 18‴ʺ angeordnet werden.

Wie insbesondere aus Fig. 9B und Fig. 9C hervorgeht, schließt sich an der distalen Spitze an das Koppelelemente 64 zunächst ein offenbarungsgemäßes Kugellager 2 an. Es folgt eine offenbarungsgemäße Hülse, welche beispielsweise wie die Hülse 18" ausgebildet sein kann (wobei der Isolator 42 auch weggelassen sein kann). Als Nächstes ist ein Kugellager 2 vorgesehen, an welches sich wiederum eine offenbarungsgemäße Hülse anschließt. Diese Hülse ist ähnlich wie die Hülse 18 (mit zusätzlichen elektrischen Kontakten 46) bzw. ähnlich wie die Hülse 18‴ (ohne Ausleseantenne 48) ausgebildet. Es folgen ein Kugellager 2, eine Hülse 18" (mit oder ohne Isolator 42), ein Kugellager 2, eine Hülse 18', ein Kugellager 2 und eine Hülse 18.

Für die elektrische Verbindung/ Konnektierung zwischen Kugellager 2, 2' und Hülse 18, 18', 18", 18‴, 18"", 18‴ʺ gehen die hervorstehenden axialen Enden 16 der an den Kugellagern 2, 2' vorgesehenen Signalleitungen 14 eine Steckverbindung mit den Hülsen 18, 18', 18", 18‴, 18"", 18‴ʺ ein. Beispielsweise können die axialen Enden 16 in Aussparungen 44 oder in Rillen 32 eingesteckt werden.

Wie insbesondere aus Fig. 9D hervorgeht, welche die Anordnung von Kugellagern 2, 2' und Hülsen 18, 18', 18", 18‴, 18ʺʺ, 18‴ʺ (ohne Außenrohr 40) zeigt, können durch ein Aneinanderstecken eine beliebige Anzahl von Kugellagern 2, 2' und Hülsen 18, 18', 18", 18‴, 18"", 18‴ʺ kombiniert werden.

### Bezugszeichenliste

- 2, 2': Kugellager
- 4: chirurgisches Instrument
- 6: Innenring
- 8: Außenring
- 10: Kugel
- 11: Käfig
- 12: Bohrung
- 14: Signalleitung
- 16: axiale Enden (Signalleitung)
- 18 bis 18‴ʺ: Hülse
- 20: erstes axiales Ende (Kugellager)
- 22: zweites axiales Ende (Kugellager)
- 24: Außenmantelfläche
- 26: Innenmantelfläche
- 28: erstes axiales Ende (Hülse)
- 30: zweites axiales Ende (Hülse)
- 32: Rille
- 34: Signalleitung (Außenmantelfläche)
- 36: Signalleitung (Innenmantelfläche)
- 38: (Mikro-) Bohrung
- 40: Außenrohr (chirurgisches Instrument)
- 42: Isolator
- 44: Aussparung
- 46: elektrischer Kontakt (Innenseite)
- 47: elektrischer Kontakt (Außenseite)
- 48: Ausleseantenne
- 50: Sensor
- 52: äußere Hülse
- 54: mittlere Hülse
- 56: innere Hülse
- 58: Verbindungsbereich
- 60: distaler Bereich
- 62: proximaler Bereich
- 64: Koppelelement

## Patentansprüche

1. Chirurgisches Instrument (4), insbesondere Fräshandstück, mit: zumindest einem Wälzlager (2, 2'), insbesondere Kugellager, welches zur Weiterleitung bzw. Übertragung von elektrischen Signalen eingerichtet ist und dazu zumindest eine in das Wälzlager (2, 2') integrierte Wälzlagersignalleitung (14) bzw. Wälzlagersignalbahn aufweist.

2. Chirurgisches Instrument (4) nach Anspruch 1, **dadurch gekennzeichnet, dass** zumindest eine Wälzlagersignalleitung (14), insbesondere Wälzlagersignallitze, vorgesehen ist, welche in eine in dem Wälzlager (2, 2') vorgesehene Bohrung (12), die sich über eine gesamte axiale Länge des Wälzlagers (2, 2') erstreckt, eingesetzt ist und bevorzugt axial fixiert ist.

3. Chirurgisches Instrument (4) nach Anspruch 2, **dadurch gekennzeichnet, dass** die Wälzlagersignalleitung (14) in axialer Richtung des Wälzlagers (2, 2') hervorsteht, so dass die Wälzlagersignalleitung (14) zur Kontaktierung bzw. Steckverbindung mit einem anderen Bauteil, insbesondere mit einer Hülse (18, 18', 18", 18‴, 18"", 18‴ʺ), eingerichtet ist.

4. Chirurgisches Instrument (4) nach einem der Ansprüche 1 bis 3, ferner mit zumindest einer Hülse (18, 18', 18", 18‴, 18"", 18‴ʺ), welche zur Weiterleitung bzw. Übertragung von elektrischen Signalen eingerichtet ist und dazu zumindest eine in die Hülse (18, 18', 18", 18‴, 18"", 18‴ʺ) integrierte Hülsensignalleitung (34, 36) bzw. Hülsensignalbahn aufweist.

5. Chirurgisches Instrument (4) nach Anspruch 4, **dadurch gekennzeichnet, dass** die Hülse (18, 18', 18", 18‴, 18ʺʺ, 18‴ʺ) zur Weiterleitung bzw. Übertragung von elektrischen Signalen in einer Axialrichtung zwischen einem ersten axialen Ende (28) und einem zweiten axialen Ende (30) der Hülse (18, 18', 18", 18‴, 18"", 18‴ʺ) und/oder in einer Radialrichtung zwischen einer Innenmantelfläche (26) und einer Außenmantelfläche (24) der Hülse (18, 18', 18", 18‴, 18"", 18‴ʺ) eingerichtet ist.

6. Chirurgisches Instrument (4) nach Anspruch 4 oder 5, **dadurch gekennzeichnet, dass** eine Außenmantelfläche (24) der Hülse (18, 18', 18", 18‴, 18"", 18‴ʺ) zumindest eine Rille (32) aufweist, welche sich über eine gesamte axiale Länge der Hülse (18, 18', 18", 18‴, 18ʺʺ, 18‴ʺ) erstreckt, und in der Rille (32) die Hülsensignalleitung (34) bzw. Hülsensignalbahn vorgesehen ist.

7. Chirurgisches Instrument (4) nach Anspruch 6, **dadurch gekennzeichnet, dass** die Hülsensignalleitung (34) bzw. Hülsensignalbahn bezüglich der Außenmantelfläche (24) der Hülse (18, 18', 18", 18‴, 18"", 18‴ʺ) nach innen versetzt ist, so dass die Hülsensignalleitung (34) bzw. Hülsensignalbahn nur in einem unteren Bereich der Rille (32) vorgesehen ist, wobei bevorzugt über der Hülsensignalleitung (34) bzw. Hülsensignalbahn ein Isolator (42) angeordnet ist.

8. Chirurgisches Instrument (4) nach einem der Ansprüche 4 bis 7, **dadurch gekennzeichnet, dass** eine Innenmantelfläche (26) der Hülse (18, 18‴, 18"", 18‴ʺ) zumindest eine Hülsensignalleitung (36) bzw. Hülsensignalbahn aufweist.

9. Chirurgisches Instrument (4) nach einem der Ansprüche 4 bis 8, **dadurch gekennzeichnet, dass** eine an einer Innenmantelfläche (26) der Hülse (18, 18‴, 18"", 18‴ʺ) vorgesehene Hülsensignalleitung (36) bzw. Hülsensignalbahn elektrisch leitend mit einer an einer Außenmantelfläche (24) der Hülse (18, 18‴, 18"", 18‴ʺ) vorgesehenen Hülsensignalleitung (36) bzw. Hülsensignalbahn verbunden ist.

10. Chirurgisches Instrument (4) nach einem der Ansprüche 4 bis 9, **dadurch gekennzeichnet, dass** ein elektrischer Kontakt (46, 47) und/oder eine Ausleseantenne (48) elektrisch leitend mit der Hülsensignalleitung (34, 36) bzw. Hülsensignalbahn verbunden ist/sind.

11. Chirurgisches Instrument (4) nach einem der Ansprüche 4 bis 10, **dadurch gekennzeichnet, dass** die Hülse (18‴ʺ) aus einer Vielzahl von ineinander gesetzten Hülsen (52, 54, 56) besteht.

12. Chirurgisches Instrument (4) nach einem der Ansprüche 4 bis 11, **dadurch gekennzeichnet, dass** die Hülse (18, 18', 18", 18‴, 18"", 18‴ʺ) hergestellt ist durch:
Fertigen der Hülse (18, 18', 18", 18‴, 18"", 18‴ʺ) aus einem nichtleitenden Material; und
Bereitstellen einer leitenden Signalleitung (34, 36) bzw. Signalbahn an einer Außenmantelfläche (24) und/oder einer Innenmantelfläche (26) der Hülse durch Metallisieren und gegebenenfalls Beschichten mit einem leitenden Material.

13. Chirurgisches Instrument (4) nach einem der Ansprüche 4 bis 12, **dadurch gekennzeichnet, dass** das Wälzlager (2, 2') und die Hülse (18, 18', 18", 18‴, 18"", 18‴ʺ) aneinander angrenzend in dem chirurgischen Instrument (4) angeordnet sind, derart, dass die zumindest eine Wälzlagersignalleitung (14) bzw. Wälzlagersignalbahn des Wälzlagers (2, 2') mit der zumindest einen Hülsensignalleitung (34, 36) bzw. Hülsensignalbahn der Hülse (18, 18', 18", 18‴, 18"", 18‴ʺ) über eine Steckverbindung verbunden ist, so dass das chirurgische Instrument (4) zur Signalweiterleitung bzw. Signalübertragung zwischen dem Wälzlager (2, 2') und der Hülse (18, 18', 18", 18‴, 18ʺʺ, 18‴ʺ) eingerichtet ist.

14. Verfahren zur Herstellung eines Wälzlagers (2, 2'), insbesondere eines Wälzlagers (2, 2') eines chirurgischen Instruments (4) nach einem der Ansprüche 1 bis 13, mit den Schritten:
Bereitstellen zumindest einer in Axialrichtung des Wälzlagers (2, 2') verlaufenden, durchgehenden Bohrung (12), insbesondere in einem Außenring (8) des Wälzlagers, insbesondere durch Mikrolaserbohren;
Einsetzen einer Signalleitung (14), insbesondere Signallitze, in die Bohrung (12); und
Bereitstellen einer axialen Fixierung der Signalleitung (14) in der Bohrung (12).

15. Verwendung eines Wälzlagers (2, 2'), welches zur Weiterleitung bzw. Übertragung von elektrischen Signalen eingerichtet ist und dazu zumindest eine in das Wälzlager (2, 2') integrierte Wälzlagersignalleitung (14) bzw. Wälzlagersignalbahn aufweist, in einem chirurgischen Instrument (4).

## Claims

1. A surgical instrument (4), in particular a hend-held milling cutter, comprising: at least one roller bearing (2, 2'), in particular a ball bearing, configured for forwarding or transmitting electrical signals, and having at least one roller bearing signal line (14) or roller bearing signal path integrated in the roller bearing (2, 2').

2. The surgical instrument (4) according to claim 1, **characterized in that** at least one roller bearing signal line (14), particularly a roller bearing signal strand, is provided that preferably is axially fixed and inserted into a bore (12) extending over an entire axial length of the roller bearing (2, 2').

3. The surgical instrument (4) according to claim 2, **characterized in that** the roller bearing signal line (14) protrudes in an axial direction of the roller bearing (2, 2') so that the roller bearing signal line (14) is configured for contacting or plug connection with another component, in particular with a sleeve (18, 18', 18", 18‴, 18ʺʺ, 18‴ʺ).

4. The surgical instrument according to one of claims 1 to 3, further comprising at least one sleeve (18, 18', 18", 18‴, 18ʺʺ, 18‴ʺ) configured for forwarding or transmitting electrical signals and having at least one sleeve signal line (34, 36) or sleeve signal path integrated in the sleeve (18, 18', 18", 18‴, 18ʺʺ, 18‴ʺ).

5. The surgical instrument (4) according to claim 4, **characterized in that** the sleeve (18, 18', 18", 18‴, 18ʺʺ, 18‴ʺ) is configured for forwarding or transmitting electrical signals in an axial direction between a first axial end (28) and a second axial end (30) of the sleeve (18, 18', 18", 18‴, 18ʺʺ, 18‴ʺ) and/or in a radial direction between an inner shell surface (26) and an outer shell surface (24) of the sleeve (18, 18', 18", 18‴, 18"", 18‴ʺ).

6. The surgical instrument (4) according to claims 4 or 5, **characterized in that** an outer shell surface (24) of the sleeve (18, 18', 18", 18‴, 18"", 18‴ʺ) has at least one groove (32) extending over an entire axial length of the sleeve (18, 18', 18", 18‴, 18ʺʺ, 18‴ʺ), and the sleeve signal line (34) or sleeve signal path is provided in the groove (32).

7. The surgical instrument (4) according to claim 6, **characterized in that** the sleeve signal line (34) or sleeve signal path is shifted inwards with respect to the outer shell surface (24) of the sleeve (18, 18', 18", 18‴, 18ʺʺ, 18‴ʺ), so that the sleeve signal line (34) or sleeve signal path is provided only in a lower region of the groove (32), wherein an insulator (42) is arranged preferably above the sleeve signal line (34) or sleeve signal path.

8. The surgical instrument (4) according to any of claims 4 to 7, **characterized in that** an inner shell surface (26) of the sleeve (18, 18', 18", 18‴, 18ʺʺ, 18‴ʺ) comprises at least one sleeve signal line (36) or sleeve signal path.

9. The surgical instrument (4) according to any of claims 4 to 8, **characterized in that** a sleeve signal line (36) or sleeve signal path provided on an inner shell surface (26) of the sleeve (18, 18‴, 18ʺʺ, 18‴ʺ) is connected in an electrically conducting manner to a sleeve signal line (36) or sleeve signal path provided on an outer shell surface (24) of the sleeve (18, 18‴, 18ʺʺ, 18‴ʺ).

10. The surgical instrument (4) according to any of claims 4 to 9, **characterized in that** an electric contact (46, 47) and/or a read-out antenna (48) is/are connected to the sleeve signal line (34, 36) or sleeve signal path in an electrically conducting manner.

11. The surgical instrument (4) according to any of claims 4 to 10, **characterized in that** the sleeve (18‴ʺ) consists of a large number of sleeves (52, 54, 56) inserted into each other.

12. The surgical instrument (4) according to any of claims 4 to 11, **characterized in that** the sleeve (18, 18', 18", 18‴, 18ʺʺ, 18‴ʺ) is produced by:
Manufacturing the sleeve (18, 18', 18", 18‴, 18ʺʺ, 18‴ʺ) from a non-conductive material, and
Providing a conductive signal line (34, 36) or signal path on an outer shell surface (24) and/or an inner shell surface (26) of the sleeve, by metallizing and possibly coating with a conductive material.

13. The surgical instrument (4) according to any of claims 4 to 12, **characterized in that** the roller bearing (2, 2') and the sleeve (18, 18', 18", 18‴, 18ʺʺ, 18‴ʺ) are arranged adjacent to each other in the surgical instrument (4) such that the at least one roller bearing signal line (14) or roller bearing signal path of the roller bearing (2, 2') is connected to the at least one sleeve signal line (34, 36) or sleeve signal path of the sleeve (18, 18', 18", 18‴, 18ʺʺ, 18‴ʺ) by a plug connection, such that the surgical instrument (4) is configured for signal forwarding or signal transmission between the roller bearing (2, 2') and the sleeve (18, 18', 18", 18‴, 18ʺʺ, 18‴ʺ).

14. A method for manufacturing a roller bearing (2, 2'), in particular of a roller bearing (2, 2') of a surgical instrument (4) according to any of claims 1 to 13, comprising the steps of:
Providing at least one continuous bore (12) running in an axial direction of the roller bearing (2, 2'); in particular in an outer ring (8) of the roller bearing, especially by microlaser drilling;
Inserting a signal line (14), in particular a signal strand, into the bore (12); and
Providing an axial fixation of the signal line (14) in bore (12).

15. Use of a roller bearing (2, 2') configured for forwarding or transmitting electrical signals and comprising for this purpose a roller bearing signal line (14) or roller bearing signal path integrated in the roller bearing (2, 2') in a surgical instrument (4).

## Revendications

1. Instrument chirurgical (4), en particulier pièce à main de fraisage, avec : au moins un palier à roulement (2, 2'), en particulier un palier à billes, lequel est conçu pour la transmission ou le transfert de signaux électriques et présente à cet effet au moins une ligne de signalisation de palier à roulement (14) ou une voie de signalisation de palier à roulement intégrée dans le palier à roulement (2, 2').

2. Instrument chirurgical (4) selon la revendication 1, **caractérisé en ce qu'**est prévue au moins une ligne de signal de palier à roulement (14), en particulier un toron de signal de palier à roulement, laquelle est insérée dans un alésage (12) prévu dans le palier à roulement (2, 2'), qui s'étend sur toute une longueur axiale du palier à roulement (2, 2'), et est de préférence fixée axialement.

3. Instrument chirurgical (4) selon la revendication 2, **caractérisé en ce que** la ligne de signal de palier à roulement (14) fait saillie dans la direction axiale du palier à roulement (2, 2'), de sorte que la ligne de signal de palier à roulement (14) est agencée pour établir un contact ou une connexion enfichable avec un autre composant, en particulier avec une douille (18, 18', 18", 18‴, 18"", 18‴ʺ).

4. Instrument chirurgical (4) selon l'une quelconque des revendications 1 à 3, comprenant en outre au moins une douille (18, 18', 18", 18‴, 18"", 18‴ʺ) qui est agencée pour la transmission ou le transfert de signaux électriques et qui présente à cet effet au moins une ligne de signal de douille (34, 36) ou une voie de signal de douille intégrée dans la douille (18, 18', 18", 18"", 18‴ʺ).

5. Instrument chirurgical (4) selon la revendication 4, **caractérisé en ce que** la douille (18, 18', 18", 18‴, 18"", 18‴ʺ) est destinée à la transmission ou au transfert de signaux électriques dans une direction axiale entre une première extrémité axiale (28) et une seconde extrémité axiale (30) de la douille (18, 18', 18", 18‴, 18"", 18‴ʺ) et/ou dans une direction radiale entre une surface d'enveloppe intérieure (26) et une surface d'enveloppe extérieure (24) de la douille (18, 18', 18", 18‴, 18"", 18‴ʺ).

6. Instrument chirurgical (4) selon la revendication 4 ou 5, **caractérisé en ce qu'**une surface d'enveloppe extérieure (24) de la douille (18, 18', 18", 18‴, 18"", 18‴ʺ) présente au moins une rainure (32) qui s'étend sur toute une longueur axiale de la douille (18, 18', 18", 18‴, 18"", 18‴ʺ), et la ligne de signal de douille (34) ou la voie de signal de douille est prévue dans la rainure (32).

7. Instrument chirurgical (4) selon la revendication 6, **caractérisé en ce que** la ligne de signal de douille (34) ou la voie de signal de douille est décalée vers l'intérieur par rapport à la surface d'enveloppe extérieure (24) de la douille (18, 18', 18", 18‴, 18"", 18‴ʺ), de sorte que la ligne de signal de douille (34) ou la voie de signal de douille est située dans une portion inférieure de la rainure (32), dans lequel de préférence un isolant (42) est agencé au-dessus de la ligne de signal de douille (34) ou de la voie de signal de douille.

8. Instrument chirurgical (4) selon l'une quelconque des revendications 4 à 7, **caractérisé en ce qu'**une surface d'enveloppe intérieure (26) du manchon (18, 18‴, 18"", 18‴ʺ) présente au moins une ligne de signal de douille (36) ou une voie de signal de douille.

9. Instrument chirurgical (4) selon l'une quelconque des revendications 4 à 8, **caractérisé en ce qu'**une ligne de signal de douille (36) ou une voie de signal de douille prévue sur une surface d'enveloppe intérieure (26) de la douille (18, 18‴, 18"", 18‴ʺ) est reliée de manière électriquement conductrice à une ligne de signal de douille (36) ou à une voie de signal de douille prévue sur une surface d'enveloppe extérieure (24) de la douille (18, 18‴, 18"", 18‴ʺ).

10. Instrument chirurgical (4) selon l'une quelconque des revendications 4 à 9, **caractérisé en ce qu'**un contact électrique (46, 47) et/ou une antenne de lecture (48) est/sont relié(s) de manière électriquement conductrice à la ligne de signal de douille (34, 36) ou à la voie de signal de douille.

11. Instrument chirurgical (4) selon l'une quelconque des revendications 4 à 10, **caractérisé en ce que** la douille (18‴ʺ) est constituée d'une pluralité de douilles (52, 54, 56) emboîtées les unes dans les autres.

12. Instrument chirurgical (4) selon l'une quelconque des revendications 4 à 11, **caractérisé en ce que** la douille (18, 18', 18", 18‴, 18"", 18‴ʺ) est réalisée comme suit :
fabrication de la douille (18, 18', 18", 18‴, 18"", 18‴ʺ) à partir d'un matériau non conducteur ; et
préparation d'une ligne de signal (34, 36) ou d'une voie de signal conductrice sur une surface d'enveloppe extérieure (24) et/ou une surface d'enveloppe intérieure (26) de la douille par métallisation et éventuellement par revêtement avec un matériau conducteur.

13. Instrument chirurgical (4) selon l'une quelconque des revendications 4 à 12, **caractérisé en ce que** le palier à roulement (2, 2') et la douille (18, 18', 18", 18"", 18‴ʺ) sont agencés de manière adjacente l'un à l'autre dans l'instrument chirurgical (4), de sorte que l'au moins une ligne de signal de palier à roulement (14) ou voie de signal de palier à roulement du palier à roulement (2, 2') est reliée à l'au moins une ligne de signal de douille (34, 36) ou voie de signal de douille de la douille (18, 18', 18", 18‴, 18"", 18‴ʺ) par le biais d'une connexion enfichable, de sorte que l'instrument chirurgical (4) est conçu pour la transmission de signaux ou le transfert de signaux entre le palier à roulement (2, 2') et la douille (18, 18', 18", 18"", 18"", 18‴ʺ).

14. Procédé de fabrication d'un palier à roulement (2, 2'), en particulier d'un palier à roulement (2, 2') d'un instrument chirurgical (4) selon l'une quelconque des revendications 1 à 13, comprenant les étapes suivantes :
préparation d'au moins un alésage (12) traversant s'étendant dans la direction axiale du palier à roulement (2, 2'), en particulier dans une bague extérieure (8) du palier à roulement, en particulier par micro-perçage au laser ;
insertion d'une ligne de signal (14), en particulier d'un fil de signal, dans l'alésage (12) ; et
préparation d'une fixation axiale de la ligne de signal (14) dans l'alésage (12).

15. Utilisation dans un instrument chirurgical (4) d'un palier à roulement (2, 2') qui est conçu pour la transmission ou le transfert de signaux électriques et qui présente à cet effet au moins une ligne de signal de palier à roulement (14) ou une voie de signal de palier à roulement intégrée dans le palier à roulement (2, 2').
